# EUROPÄISCHE PATENTANMELDUNG

(11) **EP 2 452 963 A1**
(43) Veröffentlichungstag der Anmeldung: **16.05.2012**
(21) Anmeldenummer: 10190823.4
(22) Anmeldetag: 11.11.2010
(51) Int. Cl.: C08G 59/40, C08G 65/26

(54) **Kondensationsprodukte aus aminofunktionellen Polymeren**

(71) Anmelder: Sika Technology AG, 6340 Baar (CH)
(72) Erfinder: Finter, Jürgen, 8037, Zürich (CH); Gerber, Ulrich, 8142, Uitikon-Waldegg (CH); Kasemi, Edis, 8046, Zürich (CH); Kramer, Andreas, 8006, Zürich (CH)
(74) Vertreter: Sika Patent Attorneys

(57) **Zusammenfassung**

Beschrieben wird ein Kondensationsprodukt aus mindestens einem (Hydroxymethyl)phenol und mindestens einem Polyoxyalkylendiamin, ein Verfahren zu dessen Herstellung und dessen Verwendung in der Härtung von Epoxidharzsystemen.

## Beschreibung

Die vorliegende Erfindung betrifft neuartige Mannich-analoge Kondensationsprodukte sowie ein neues Verfahren zur deren Herstellung. Weiterer Gegenstand der vorliegenden Erfindung ist die Verwendung dieser Kondensationsprodukte als Beschleuniger in der Härtung von Ein- und Zweikomponenten-Epoxidharz-Klebstoffen.

### Stand der Technik

Ein Epoxidharz besteht aus Polyetherpolymeren, die je nach Reaktionsführung unter Zugabe geeigneter Härter einen duroplastischen Kunststoff von hoher Festigkeit und chemischer Beständigkeit ergeben. Werden Epoxidharz und Härter gemischt, erfolgt je nach Zusammensetzung und Temperatur üblicherweise innerhalb von wenigen Minuten bis einigen Stunden die Aushärtung des ursprünglich viskosen Gemisches. Als Härter für Epoxidharze sind unterschiedliche Systeme kommerziell erhältlich, wie beispielsweise Härter auf Amin-, Amid- und Anhydridbasis (beispielsweise unter der Bezeichnung Epikure^{®} von Hexion Speciality Chemicals) oder Härter auf Basis von Polyetheraminen (beispielsweise unter der Bezeichnung Jeffamine^{®} von Huntsman).

Als Härter bzw. Beschleuniger können auch Mannichbasen aus primären Aminen, Formaldehyd und Phenolen eingesetzt werden (vgl. u.a EP 0 253 339 A). Ferner beschreibt auch die WO 00/15687 einen Mannichbasen-Beschleuniger, welcher durch Transaminierung einer Mannichbase mit einem Amin hergestellt wird.

Ein Nachteil entsprechender herkömmlicher Mannichbasen liegt in der Verwendung von Phenol als Ausgangsmaterial, da die erhaltenen Mannichbasen häufig noch nicht umgesetztes Phenol umfassen. Aufgrund der Toxizität von freiem Phenol sind auf Phenol basierende Mannichbasen für viele Anwendungsbereiche daher nicht einsetzbar. Deshalb sind große Bestrebung getätigt worden, Phenol-freie Mannichbasen herzustellen. So wurden beispielsweise Mannichbasen auf Basis von Nonylphenol oder p-tert.-Butylphenol oder Cardanol entwickelt (vgl. US 6,262,148). Auch entsprechende kommerzielle Produkte, wie Aradur^{®} 3442 von Huntsman Advanced Materials Inc., sind bekannt.

Ein weiterer Nachteil bei der Verwendung von herkömmlichen Mannichbasen besteht darin, dass diese unter Verwendung von Formaldehyd hergestellt werden. Formaldehyd wird unter anderem für die Entstehung von Allergien, Haut-, Atemwegs- und Augenreizungen verantwortlich gemacht. Demgemäß besteht Bedarf an der Bereitstellung von Mannich-analogen Kondensationsprodukten, welche unter Vermeidung von Formaldehyd herstellbar sind.

Ein weiterer großer Nachteil bekannter herkömmlicher Mannich-Härter ist die hohe Viskosität, die bei deren Herstellung aufgrund der Bildung von Oligomeren und Nebenprodukten entsteht. So bilden sich durch die Umsetzung von Phenolen mit Formaldehyd in einer herkömmlichen Mannichbasensynthese unter basischen Bedingungen hochmolekulare und verhältnismäßig hochviskose Resole aus. Wenn diese hochmolekularen und verhältnismäßigen hochviskosen Resole beispielsweise mit polymeren Polyaminen, die per se ebenfalls eine höhere Viskosität aufweisen, zu herkömmlichen Mannichbasen umgesetzt werden sollen, gelingt dieses aufgrund der hohen Viskosität der resultierenden Reaktionsmischung im Allgemeinen nicht. Daher sind herkömmliche Mannichbasen aus Phenolen, Formaldehyd und polymeren Polyaminen im Allgemeinen nicht zugänglich.

Bei der Herstellung von herkömmlichen Mannichbasen-Härtern wird üblicherweise mit einem großen Überschuss an Amin gearbeitet, um das Molekulargewicht und so die Viskosität niedrig zu halten. Daher werden im Allgemeinen herkömmliche Mannichbasen-Härter in Abmischung mit üblichen Polyaminen eingesetzt. Die Zumischung zusätzlicher Polyamine wirkt sich jedoch meist negativ auf die Eigenschaften der ausgehärteten Epoxidharz-Zusammensetzung aus.

Darüber hinaus sind die Verfahren zur Herstellung bekannter Mannichbasen sehr aufwändig und schwierig zu führen, insbesondere dann, wenn die Ausbildung von hochmolekularen Kondensationsprodukten möglichst verhindert werden soll. So offenbart beispielsweise die EP 1 475 411 A ein zweistufiges Herstellverfahren zur Herstellung von Mannichbasen basierend auf m-Kresol oder 3,5-Xylenol und Polyaminen, bei dem vorzugsweise ein tertiäres Amin verwendet wird. Ebenso wird ein zweistufiges Herstellverfahren von Mannichbasen in der EP 1 475 412 A offenbart, wobei diese aus Phenolen wie m-Kresol, 3,5-Xylenol oder Resorcin mit Polyaminen vorzugsweise unter Verwendung von tertiären Aminen gewonnen werden. Derartige zweistufige Verfahren sind mit zusätzlichem Aufwand verbunden und verteuern die Herstellung der Mannichbasen.

Ausgehend von diesem Stand der Technik stellt sich die vorliegende Erfindung die Aufgabe, alternative Beschleuniger für Epoxidharze oder Härter für Epoxidharze und Polyurethane bereitzustellen, welche die vorliegenden Nachteile vorzugsweise nicht aufweisen.

Insbesondere stellt sich die vorliegende Erfindung die Aufgabe, Beschleuniger für Epoxidharze oder Härter für Epoxidharze und Polyurethane bereitzustellen, welche kein freies Phenol enthalten.

Ferner stellt sich die vorliegende Erfindung die Aufgabe, Beschleuniger für Epoxidharze oder Härter für Epoxidharze und Polyurethane bereitzustellen, welche kein Formaldehyd enthalten.

Bei der Herstellung entsprechender Beschleuniger sollte daher vorzugsweise auf die Verwendung von Phenolen und Formaldehyd verzichtet werden.

Des Weiteren stellt sich die vorliegende Erfindung insbesondere die Aufgabe, Beschleuniger für Epoxidharze oder Härter für Epoxidharze und Polyurethane bereitzustellen, welche keine oligomeren Nebenprodukte aufweisen.
,Daher sollten die Beschleuniger auf einfache Weise ohne die Bildung von unerwünschten hochmolekularen Kondensationsprodukten erhältlich sein.

### Darstellung der Erfindung

Gelöst werden diese Aufgaben durch ein Kondensationsprodukt, welches aus mindestens einem (Hydroxymethyl)phenol der allgemeinen Formel (I) 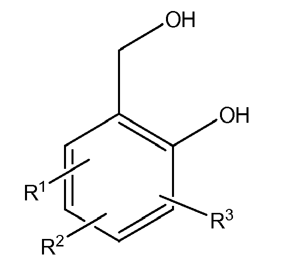 in welcher:
R¹ gleich Wasserstoff oder -CH₃ ist;
R² gleich Wasserstoff oder -CH₂OH ist; und
R³ gleich Wasserstoff oder -CH₃ ist,
und mindestens einem Polyoxyalkylendiamin aufgebaut wird.

Die erfindungsgemäßen Kondensationsprodukte eignen sich als Beschleuniger für Epoxidharze sowie als Härter für Epoxidharze oder Polyurethane.

Insbesondere eignen sich diese erfindungsgemäßen Kondensationsprodukte zur Beschleunigung der Aushärtung von Epoxidharzklebstoffen, zur Verbesserung der Haftung von Epoxidharzklebstoffen und/oder Schälfestigkeit von Epoxidharzklebstoffen, wobei aufgrund der Verwendung spezieller Phenol- und Aminkomponenten die aus dem Stand der Technik bekannten Nachteile im Wesentlichen vermieden werden. Insbesondere umfasst das erfindungsgemäße Kondensationsprodukt kein freies Phenol und keine oligomeren Nebenprodukte.

Da im Gegensatz zu der herkömmlichen Mannichbasensynthese im Rahmen der vorliegenden Erfindung auf die Verwendung von Formaldehyd verzichtet wird, sind die erfindungsgemäß resultierenden Kondensationsprodukte auch frei von Formaldehyd. Die erfindungsgemäßen Kondensationsprodukte sind aus einfach zugänglichen und kostengünstigen Rohstoffen über ein einfaches Herstellungsverfahren, welches weiter unten beschrieben wird und ebenfalls Gegenstand der vorliegenden Erfindung ist, zugänglich. Die erfindungsgemäßen Kondensationsprodukte zeichnen sich insbesondere durch ein exzellentes Härtungsverhalten von Epoxidharz-Klebstoffen aus.

Im Folgenden werden bevorzugte Ausgestaltungen des erfindungsgemäßen Kondensationsprodukts näher beschrieben.

Die vorliegende Erfindung betrifft ein Kondensationsprodukt, welches aus mindestens einem (Hydoxymethyl)phenol als Komponente (A) und mindestens einem Polyoxyalkylendiamin als Komponente (B) hergestellt wird.

### Komponente (A) - (Hydoxymethyl)phenol

Unter einem (Hydroxymethyl)phenol wird im Rahmen der vorliegenden Erfindung ein aromatisches Phenol verstanden, welches in ortho-, meta- oder para-Position zur phenolischen OH-Gruppe mindestens einen Methylolsubstituenten trägt. Solche Phenole sind kommerziell erhältlich.

Im Rahmen der vorliegenden Erfindung können die Phenole vorzugsweise eine oder zwei Methylolgruppe tragen.

Geeignete Beispiele sind 2-(Hydroxymethyl)phenol (Salicylalkohol), 3-(Hydroxymethyl)phenol, 4-(Hydroxymethyl)phenol sowie 2,6-Di(hydroxymethyl)-4-methylphenol.

Das in dem erfindungsgemäßen Kondensationsprodukt verwendete (Hydroxymethyl)phenol ist ein mit einer Methylolgruppe in ortho-Position substituiertes Phenol, welches gegebenenfalls weitere Substituenten aufweist, und entspricht der allgemeinen Formel (I) 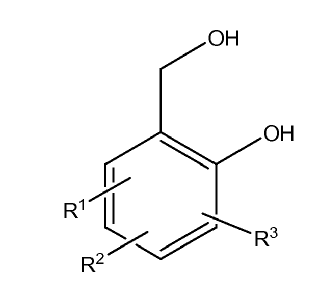 in welcher:
R¹ gleich Wasserstoff oder -CH₃ ist;
R² gleich Wasserstoff oder -CH₂OH ist; und
R³ gleich Wasserstoff oder -CH₃ ist.

Selbstverständlich können im Rahmen der vorliegenden Erfindungen zur Herstellung der erfindungsgemäßen Kondensationsprodukte auch Mischungen solcher (Hydroxymethyl)phenole verwendet werden.

Die Auswahl der eingesetzten (Hydroxymethyl)phenole beeinflusst stark die Eigenschaften der resultierenden Kondensationsprodukte und damit der unter Verwendung dieser Kondensationsprodukte als Härter bzw. Beschleuniger resultierenden EpoxidSysteme.

### Komponente (B) - Polyoxyalkylendiamin

Das erfindungsgemäße Kondensationsprodukt wird ferner aus mindestens einem Polyoxyalkylendiamin aufgebaut. Im Rahmen der vorliegenden Erfindung wird unter einem Polyoxyalkylendiamin eine Verbindung verstanden, welche terminal jeweils zwei primäre Aminfunktionen aufweist, die durch ein Polyoxyalkylen-Rückgrat miteinander verbunden sind.

Das in dem erfindungsgemäßen Kondensationsprodukt verwendete Polyoxyalkylendiamin weist dabei vorzugsweise Einheiten auf Basis von Propylenoxid oder Ethylenoxid und Propylenoxid auf.

Des Weiteren ist es bevorzugt, wenn das Polyoxyalkylendiamin ein Molekulargewicht in einem Bereich von 220 bis 10.000 g/mol aufweist.

In einer ersten Ausgestaltung der vorliegenden Erfindung weist das Polyoxyalkylendiamin die allgemeine Struktur (II) 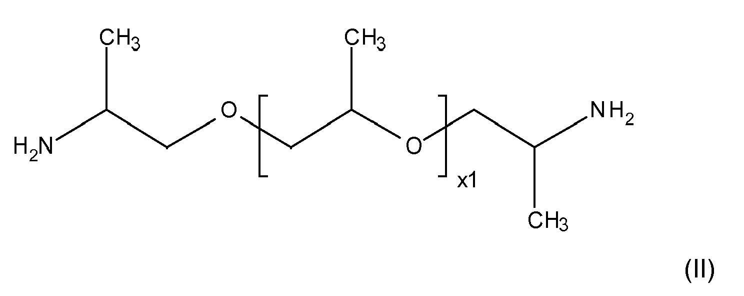 auf, in welcher X1 für einen Wert von 2 bis 70 steht.

Entsprechende Polyoxyalkylendiamine sind kommerziell erhältlich von Huntsman unter den Bezeichnungen Jeffamine^{®} D-230, D-400, D-2000 und D-4000 mit unterschiedlichen Anteilen an Propylenglykoleinheiten.

In einer weiteren Ausgestaltung der vorliegenden Erfindung weist das Polyoxyalkylendiamin die allgemeine Struktur (III) auf : 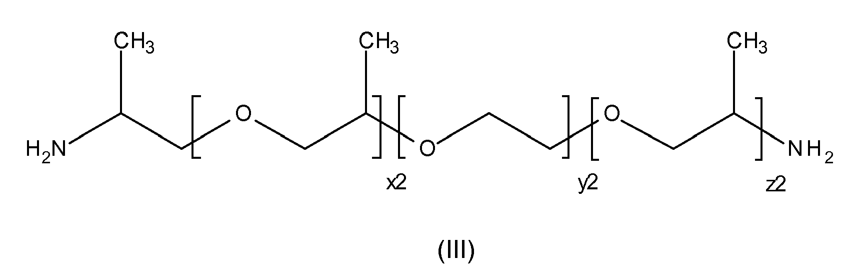 in welcher die Indizes x2, y2 und z2 die folgende Bedeutung aufweisen:
o y2 gleich 2 bis 40 und
o x2 + z2 gleich 1 bis 7.

Entsprechende Polyoxyalkylendeamine sind kommerziell erhältlich von Huntsman unter den Bezeichnungen Jeffamine^{®} HK-511 (y2 = 2; x2 + z2 = ∼ 1,2); ED-600 (y2 = ∼ 9; x2 + z2 = ∼ 3,6); ED-900 (y2 = ∼ 12,5; x2 + z2 = ∼ 6,0) und ED-2003 (y2 = ∼ 39; x2 + z2 = ∼ 6,0).

Die erfindungsgemäßen Kondensationsprodukte aus (Hydroxymethyl)phenolen und Polyoxyalkylendiaminen lassen sich aus den zuvor beschriebenen phenolischen Verbindungen und Polyoxyalkylendiaminen durch Kondensationsreaktion herstellen. Die Herstellung dieser Kondensationsprodukte ist nach üblichen Kondensationsverfahren möglich. Bevorzugt werden jedoch die Kondensationsprodukte nach dem im folgenden beschriebenen Verfahren hergestellt:

So stellt ein weiterer Aspekt der vorliegenden Erfindung ein neues Verfahren für die Herstellung von erfindungsgemäßen Kondensationsprodukten dar. Dieses erfindungsgemäße Verfahren zur Herstellung zeichnet sich dadurch aus, dass mindestens ein (Hydroxymethyl)phenol mit mindestens einem Polyoxyalkylendiamin umgesetzt wird.

Als (Hydroxymethyl)phenol kann eine wie oben beschriebene (Hydroxymethyl)-phenolverbindung verwendet werden. Insoweit wird auf die obigen Ausführungen verwiesen.

Als Polyoxyalkylendiamin kann eine wie oben beschriebene Polyoxyalkylendiaminverbindung verwendet werden. Insoweit wird ebenfalls auf die obigen Ausführungen verwiesen.

In einer bevorzugten Ausführungsform umfasst das erfindungsgemäße Verfahren die folgenden Verfahrensschritte:
(i) Vorlegen von mindestens einem (Hydroxymethyl)phenol und von mindestens einem Polyoxyalkylendiamin bei einer Temperatur von 15 bis 100 °C, bevorzugt 20 bis 90 °C, besonders bevorzugt 20 bis 70 °C, in einem Reaktionsgefäß;
(ii) Erhöhen der Temperatur auf 120 bis 190 °C, bevorzugt 120 bis 180 °C, besonders bevorzugt 120 bis 170 °C.

Die Umsetzung in dem erfindungsgemäße Verfahren, insbesondere der zuvor beschriebene Verfahrensschritt (ii), wird vorzugsweise für einen Zeitraum von 1 bis 10 Stunden, bevorzugt 1 bis 8 Stunden, besonders bevorzugt 2 bis 5 Stunden, durchgeführt.

Des Weiteren ist es bevorzugt, wenn das erfindungsgemäße Verfahren unter inerten Bedingungen, insbesondere in Gegenwart eines Inertgases, durchgeführt wird.

Vorteilhafterweise wird das mindestens eine (Hydroxymethyl)phenol mit dem mindestens einen Polyoxyalkylendiamin in einem stöchiometrischen Verhältnis von 1 zu 30, bevorzugt 1 zu 10, besonders bevorzugt 1 zu 1, umgesetzt.

Die vorliegende Erfindung betrifft ferner ein Kondensationsprodukt, welches durch das zuvor beschriebene Verfahren erhältlich ist.

Das erfindungsgemäße Kondensationsprodukt eignet sich insbesondere als Härter für eine Amin-reaktive Substanz, welche mindestens zwei Amin-reaktive funktionelle Gruppen aufweist. Als derartige Amin-reaktive funktionelle Gruppen kommen insbesondere Glycidylether- und/oder Isocyanat-Gruppen in Betracht.

In einer Ausführungsform der vorliegenden Erfindung ist die Amin-reaktive Substanz, welche mindestens zwei Amin-reaktive funktionelle Gruppen aufweist, ein Diglycidylether. Insbesondere ist es ein Diglycidylether von Bisphenol-A, Bisphenol-F oder Bisphenol-A/F. Solche Diglycidylether sind beispielsweise als Araldite® GY 250, Araldite® PY 304, Araldite® GY 282 (Huntsman) oder D.E.R.™ 331 oder D.E.R.™ 330 (Dow) oder Epikote 828 (Hexion) erhältlich.

Durch Mischen der Amin-reaktiven Substanz, welche mindestens zwei Amin-reaktive funktionelle Gruppen aufweist, mit dem erfindungsgemässen Kondensationsprodukt tritt eine Reaktion der Amingruppen des Kondensationsprodukts mit den Amin-reaktiven funktionellen Gruppen der Amin-reaktiven Substanz auf und es erfolgt eine Aushärtung.

Daher betrifft die vorliegende Erfindung insbesondere die Verwendung von mindestens einem erfindungsgemäßen Kondensationsprodukt oder mindestens einem gemäß dem oben beschriebenen Verfahren erhältlichen Kondensationsprodukt in Epoxidharzsystemen, beispielsweise im Rahmen von Epoxidharzklebstoffen und Beschichtungen auf Basis von Epoxidharzen.

Insbesondere werden die erfindungsgemäßen Kondensationsprodukte zur Beschleunigung der Aushärtung von Epoxidharzklebstoffen, zur Verbesserung der Haftung von Epoxidharzklebstoffen und/oder Schälfestigkeit von Epoxidharzklebstoffen verwendet.

Das erfindungsgemäße Kondensationsprodukt kann als solches oder in einer Zusammensetzung eingesetzt werden.

Die erfindungsgemäßen Kondensationsprodukte sowie die aus dem erfindungsgemäßen Verfahren resultierenden erfindungsgemäßen Kondensationsprodukte finden insbesondere Einsatz in Härterkomponenten von zweikomponentigen Epoxidsystemen. Die erfindungsgemäßen Kondensationsprodukte können hier direkt oder als Bestandteile der Härterkomponente eingesetzt werden.

Besonders bevorzugt werden die erfindungsgemäßen Kondensationsprodukte eingesetzt als Härter in zweikomponentigen Epoxidharz-Klebstoffen.

Die mit diesen erfindungsgemäßen Kondensationsprodukten gehärteten zweikomponentigen Epoxidsysteme und die daraus erhaltenen Produkte weisen sehr vorteilhafte Eigenschaften auf.

Wenn das erfindungsgemäße Kondensationsprodukt zur Aushärtung von Amin-reaktiven Systemen eingesetzt wird, betrifft die vorliegende Erfindung auch eine zweikomponentige Zusammensetzung, welche aus einer ersten Komponente **K1** und einer zweiten Komponente **K2** besteht. Die erste Komponente **K1** umfasst mindestens eine Amin-reaktive Verbindung mit mindestens zwei funktionellen Gruppen, welche mit Aminen reagieren können. Die zweite Komponente **K2** umfasst mindestens ein erfindungsgemäßes Kondensationsprodukt. Als Amin-reaktive Verbindungen, welche mindestens zwei funktionelle Gruppen aufweisen, die mit Aminen reagieren können, sind bereits vorgängig beschrieben worden.

Beide Komponenten **K1** und **K2** können bei Bedarf weitere dem Fachmann bekannte Inhaltsstoffe umfassen. Derartige weitere Inhaltsstoffe sind insbesondere Füllstoffe, Weichmacher, Lösungsmittel, Katalysatoren und/oder Additive.

Als Füllstoffe gelten insbesondere Russe, Kreiden, insbesondere beschichtete Kreiden, Sande, Silikate, Leichtfüllstoffe, wie Keramik- oder Glaskugeln, insbesondere Keramik- oder Glashohlkugeln, pyrogene Kieselsäuren, Flugasche, als bevorzugt.

Als Lösungsmittel werden insbesondere derartige Lösungsmittel bevorzugt, welche nicht als VOC "volatile organic compounds" eingestuft werden. Besonders bevorzugt werden höher siedende Kohlenwasserstoffe.

Als Weichmacher sind insbesondere Phthalate und Adipate, insbesondere Diisodecylphthalat (DIDP) und Dioctyladipat (DOA).

Derartige zweikomponentige Zusammensetzungen lassen sich breit einsetzen. Insbesondere bevorzugt ist deren Verwendung als Klebstoff oder Dichtstoff, insbesondere als struktureller Klebstoff. Es hat sich nämlich gezeigt, dass die mittels den erfindungsgemässen Kondensationsprodukten erzielbaren Eigenschaften insbesondere im Klebstoffbereich besonders erwünscht sind.

Nach dem Mischen der Komponenten **K1** und **K2** der beschriebenen zweikomponentigen Zusammensetzung wird der Klebstoff auf eine Substratoberfläche appliziert und mit einer weiteren Substartoberfläche gefügt. Die ausgehärtete Zusammensetzung wirkt als Klebschicht, welche befähigt ist, Kräfte zwischen den zwei Substratoberflächen des gebildeten Verbundkörpers zu übertragen.

Die zweikomponentige Zusammensetzung eignet sich aufgrund ihrer Eigenschaften speziell gut als struktureller Klebstoff im Hoch- und Tiefbau, sowie in der Industrie. Beispielsweise kann eine derartige zweikomponentige Zusammensetzung, insbesondere eine zweikomponentige Epoxidharz-Zusammensetzung, d.h. in welcher die Komponente **K1** einen Diglycidylether umfasst, als Klebstoff für das Verkleben von faserverstärkten Composites eingesetzt werden. Ein illustrierendes Beispiel hierfür ist das Verkleben von Kohlenfaser-Lamellen beim Verstärken von Bauwerken, wie Brücken.

Weiterhin können erfindungsgemässe zweikomponentige Zusammensetzungen, insbesondere eine zweikomponentige Epoxidharz-Zusammensetzung, als Kunststoffmatrix für die Herstellung von faserverstärkten Composites eingesetzt werden. So lassen sich beispielsweise Kohlen- oder Glasfasern in eine zweikomponentige Zusammensetzung einbetten und können im ausgehärteten Zustand als Faser-Composite, beispielsweise in Form einer Lamelle, zum Einsatz kommen.

Ebenso können beispielsweise Fasergewebe oder -gelege mittels einer zweikomponentigen Zusammensetzung, insbesondere mittels einer zweikomponentigen Epoxidharz-Zusammensetzung, auf ein Bauwerk appliziert werden, und dort mit dem Bauwerk zusammen ein faserverstärktes Composite bilden.

Die Viskosität des erfindungsgemäßen Kondensationsprodukts hängt dabei jeweils stark von der eingesetzten phenolischen Verbindung sowie vom eingesetzten Polyamin ab.

Besonders geeignete erfindungsgemäße Kondensationsprodukts weisen eine Viskosität bei 25 °C kleiner als 10000 mPas auf. Bevorzugte erfindungsgemäße Kondensationsprodukte weisen Viskositäten im Bereich zwischen 200 und 7000 mPas auf.

Dem Fachmann ist klar, dass bei dieser Art von Umsetzung in geringem Masse auch noch nicht reagierte Bestandteile im Endprodukt vorhanden sein können.

Die erfindungsgemäßen Kondensationsprodukte weisen keine oligomeren Verbindungen auf.

Die vorliegende Erfindung wird anhand der nachfolgenden Beispiele näher erläutert.

### Beispiele

Die im Folgenden genannten Beispiele dienen zur Veranschaulichung der Erfindung.

### 1. Herstellungsverfahren

Im Folgenden wird ein Verfahren zur Herstellung erfindungsgemäßer Kondensationsprodukte näher beschrieben:

### Kondensationsprodukt A

Ein 500 ml-Rundgefäß wird mit 20 g (0,0473 mol) Jeffamine^{®} D-400 und 14 g (0,946 mol) Salicylalkohol befüllt und unter Stickstoff am Rotationsverdampfer erwärmt. Nachdem eine homogene Mischung erhalten wurde (ungefähr 50 °C) wird ein Vakuum angelegt (∼ 20 mbar). Anschließend wird die Temperatur auf 165 °C erhöht. Die Reaktion wird nach 5 Stunden beendet, um ein braunes relativ viskoses Material ("Kondensationsprodukt A") zu erhalten. Eine LC-MS-Analyse zeigt, dass das Produkt ungefähr 45 % Produkt an dem erfindungsgemäßen zweikernigen aromatischen Kondensationsprodukt, 45 % einkerniges aromatisches Addukt und 10 % nichtreagiertes Jeffamine^{®} D-400 umfasst.

### 2. Anwendungsbeispiel

Es wurden die in Tabelle 1 aufgeführten Proben hergestellt

**Tabelle 1. Zusammensetzungen und Glasüberganngstemperatur.**

| | **Probe 1** | **Probe 2** | **Probe 3** |
|---|---|---|---|
| D.E.R.™ 331 (Dow Chemicals) [g] | 50.0 | 50.0 | 50.0 |
| Kondensationsprodukt A [g] | | 41.48 | |
| Jeffamine® D-400 (Huntsman) [g] | 26.6 | | 21.0 |
| Salicyl Alkohol (Aldrich) [g] | | | 8.5 |
| | | | |
| Tg [°C] | 39.8 | 38.6 | 19.3 |

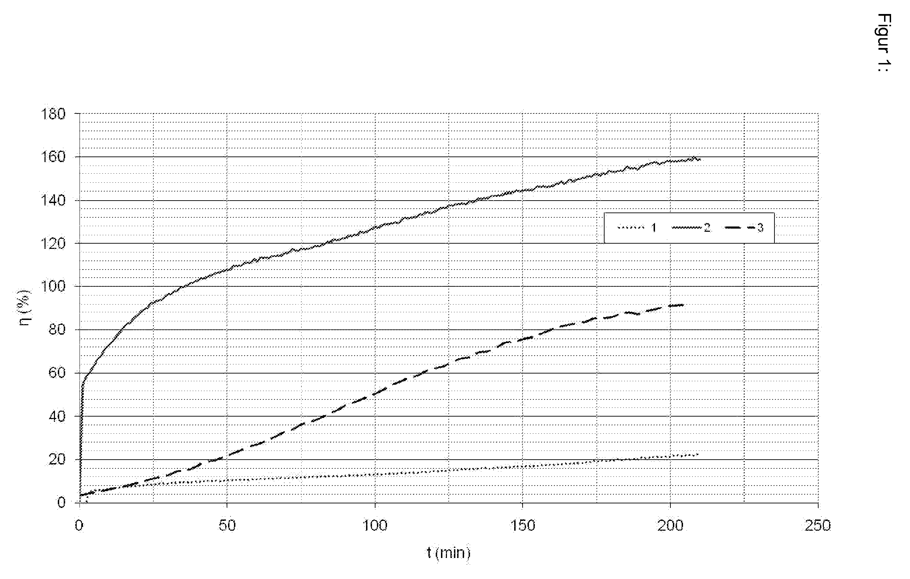

In dem Anwendungsversuch wird die zeitliche (t) Entwicklung der Viskosität (η) mit GELNORM-RVN bei Raumtemperatur untersucht. Die Viskosität wird dabei in Prozenten des maximalen Drehmoments angegeben. Die Ergebnisse sind in der Figur 1 dargestellt, in welcher (1) für die Probe 1 (Vergleichsbeispiel), (2) für die Probe 2 (erfindungsgemäßes Beispiel) und (3) für die Probe 3 (Vergleichsbeispiel) steht. Man beobachtet eine signifikante Beschleunigung der Härtung mit dem erfindungsgemäßen Kondensationsprodukt A aus Jeffamine^{®} D-400 und Salicylalkohol im Vergleich zu einer alleinigen Verwendung von Jeffamine^{®} D-400 oder zur Verwendung von Jeffamine^{®} D-400 in der Gegenwart von ungebundenem Salicylalkohol.

Die Ergebnisse sind in der Figur 1 dargestellt, in welcher (1) für die Probe 1 (Vergleichsbeispiel), (2) für die Probe 2 (erfindungsgemäßes Beispiel) und (3) für die Probe 3 (Vergleichsbeispiel) steht.

Wie der Figur 1 zu entnehmen ist, zeigt die erfindungsgemäße Anwendung (2) eine schnelle Viskositätszunahme, welche auf die fortschreitende Härtung von Bisphenol-A-diglycidylether (BADGE) zurückzuführen ist. Dieser Viskositätsanstieg ist bei (1) und (3) geringer ausgeprägt.

Der vorliegende Anwendungsversuch zeigt somit, dass die Viskosität von Bisphenol-A-diglycidylether in Gegenwart des erfindungsgemäßen Kondensationsprodukts stärker ansteigt als mit den Vergleichssubstanzen (Gegenwart von Jeffamine^{®} D-400 alleine bzw. in Gegenwart von Jeffamine^{®} D-400 und Salicylalkohol).

Diese Beobachtungen werden von DSC-Messungen bestätigt, welche 24 Stunden nach dem Härten bei Raumtemperaturen durchgeführt wurden.

Es wurde hierfür ein Gerät Mettler DSC822^{e} verwendet. 10 - 20 mg der Zusammensetzungen wurden jeweils in einen Aluminiumtiegel eingewogen. Nachdem die Probe im DSC mit einer Heizrate von 10 K / min von 25°C auf 200°C aufgeheizt wurde, wurde die Probe auf 25°C abgekühlt und anschliessend mit einer Heizrate von 10 K/min bis 100°C aufgeheizt. Die Glasübergangstemperatur (Tg) wurde mit Hilfe der DSC-Software aus der gemessenen DSC-Kurve bestimmt und in Tabelle 1 aufgeführt. Das resultierende ausgehärtete Epoxidharz weist im Fall der Verwendung von dem erfindungsgemäßen Kondensationsprodukt A als Härter (Probe 2) eine ähnliche Glasübergangstemperatur auf wie ein mit Jeffamine^{®} D-400 gehärtetes Epoxidharz (Probe 1) auf; allerdings verläuft die Härtung viel schneller.

Wie aus dem erfindungsgemäßen Beispiel ersichtlich, eignen sich die erfindungsgemäßen Kondensationsprodukte hervorragend für die Beschleunigung der Aushärtung von Epoxidharzen.

Dabei kann auf die die Verwendung von Phenol als Ausgangsmaterial verzichtet werden, was aufgrund der Toxizität von freiem Phenol von Vorteil ist. Auch auf die Verwendung von Formaldehyd kann im Rahmen der Synthese der erfindungsgemäßen Kondensationsprodukten verzichtet werden. Damit kann das erfindungsgemäße Kondensationsprodukte als Formaldehyd-freier Beschleuniger für die Aushärtung von Epoxidharzen verwendet werden.

Da im Rahmen der vorliegenden Erfindung bei der Synthese der Kondensationsprodukte auf die Verwendung von Formaldehyd verzichtet wird, kommt es auch nicht zu einer unerwünschten Bildung von Resolen, die zu einem Viskositätsanstieg bei der basischen Synthese herkömmlicher Mannichbasen führen. Damit können zur Herstellung der erfindungsgemäßen Kondensationsprodukte auch Polyamine eingesetzt werden, die per se ebenfalls eine höhere Viskosität aufweisen.

Auch eine Abmischung der erfindungsgemäßen Kondensationsprodukte mit üblichen Polyaminen zur Einstellung der Viskosität ist nicht erforderlich.

## Patentansprüche

1. Kondensationsprodukt aus mindestens einem (Hydroxymethyl)phenol der allgemeinen Formel (I) 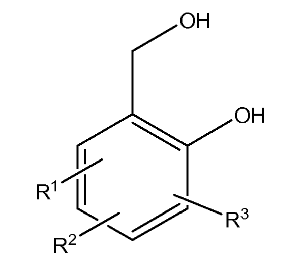 in welcher:
R¹ gleich Wasserstoff oder -CH₃ ist;
R² gleich Wasserstoff oder -CH₂OH ist; und
R³ gleich Wasserstoff oder -CH₃ ist,
und mindestens einem Polyoxyalkylendiamin.

2. Kondensationsprodukt nach Anspruch 1, **dadurch gekennzeichnet, dass** das Polyoxyalkylendiamin Einheiten auf Basis von Ethylenoxid, Propylenoxid oder Ethylenoxid und Propylenoxid umfasst.

3. Kondensationsprodukt nach Anspruch 1 oder 2, **dadurch gekennzeichnet, dass** das Polyoxyalkylendiamin der allgemeinen Struktur (II) entspricht: 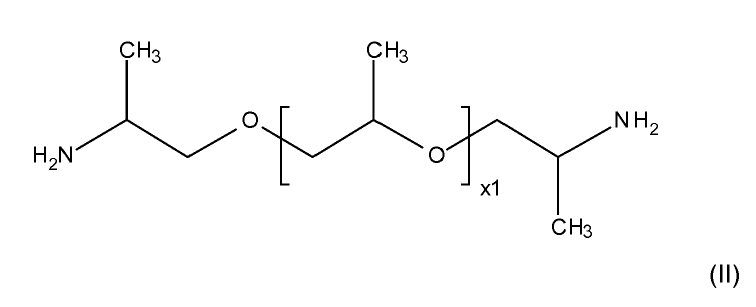 auf, in welcher X1 für einen Wert von 2 bis 70 steht.

4. Kondensationsprodukt nach Anspruch 1 oder 2, **dadurch gekennzeichnet, dass** das Polyoxyalkylendiamin der allgemeinen Struktur (III) entspricht: 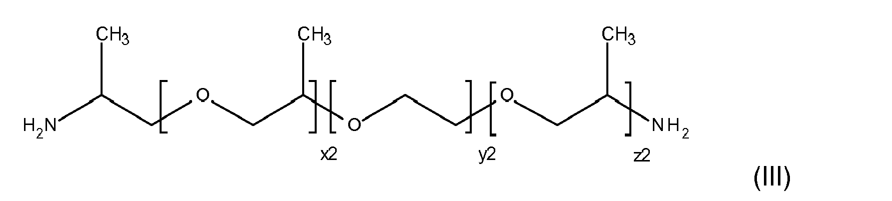 in welcher die Indizes x2, y2 und z2 die folgende Bedeutung aufweisen:
o y2 gleich 2 bis 40 und
o x2 + z2 gleich 1 bis 7.

5. Kondensationsprodukt nach einem der Ansprüche 1 bis 4, **dadurch gekennzeichnet, dass** das Polyoxyalkylendiamin ein Molekulargewicht von 220 bis 10.000 g/mol aufweist.

6. Verfahren zur Herstellung eines Kondensationsprodukts, **dadurch gekennzeichnet, dass** mindestens ein (Hydroxymethyl)phenol mit mindestens einem Polyoxyalkylendiamin umgesetzt wird.

7. Verfahren nach Anspruch 6, **dadurch gekennzeichnet, dass** ein (Hydroxymethyl)phenol gemäß der Definition in Anspruch 1 verwendet wird.

8. Verfahren nach Anspruch 6 oder 7, **dadurch gekennzeichnet, dass** ein Polyoxyalkylendiamin gemäß der Definition in einem der Ansprüche 2 bis 5 verwendet wird.

9. Verfahren nach einem der Ansprüche 6 bis 8, umfassend die folgenden Verfahrensschritte:
(i) Vorlegen von mindestens einem (Hydroxymethyl)phenol und von mindestens einem Polyoxyalkylendiamin bei einer Temperatur von 15 bis 100 °C, bevorzugt 20 bis 90 °C, besonders bevorzugt 20 bis 70 °C, in einem Reaktionsgefäß;
(ii) Erhöhen der Temperatur auf 120 bis 190 °C, bevorzugt 120 bis 180 °C, besonders bevorzugt 120 bis 170 °C.

10. Verfahren nach Anspruch 9, **dadurch gekennzeichnet, dass** das Verfahren für einen Zeitraum von 1 bis 10 Stunden, bevorzugt 1 bis 8 Stunden, besonders bevorzugt 2 bis 5 Stunden, durchgeführt wird.

11. Verfahren nach einem der Ansprüche 6 bis 10, **dadurch gekennzeichnet, dass** das mindestens eine (Hydroxymethyl)phenol mit dem mindestens einen Polyoxyalkylendiamin in einem stöchiometrischen Verhältnis von 1 zu 30, bevorzugt 1 zu 10, besonders bevorzugt 1 zu 1, umgesetzt wird.

12. Kondensationsprodukt, erhältlich nach einem Verfahren gemäß einem der Ansprüche 6 bis 11.

13. Verwendung von mindestens einem Kondensationsprodukt gemäß einem der Ansprüche 1 bis 5 oder gemäß Anspruch 12 in Epoxidharzsystemen, insbesondere in Epoxidharzklebstoffen und Epoxidharzbeschichtungen.

14. Verwendung nach Anspruch 13 zur Beschleunigung der Aushärtung von Epoxidharzklebstoffen, zur Verbesserung der Haftung von Epoxidharzklebstoffen und/oder Schälfestigkeit von Epoxidharzklebstoffen.

15. Zweikomponentige Zusammensetzung, umfassend eine ersten Komponente, welche mindestens einen Amin-reaktive Verbindung mit mindestens zwei funktionellen Gruppen, welche mit Aminen reagieren können, umfasst, und eine zweite Komponente, welche mindestens eine Kondensationsprodukt gemäss einem der Ansprüche 1 bis 5 oder gemäß Anspruch 12 umfasst.
